# EUROPEAN PATENT APPLICATION

(11) **EP 3 812 375 A1**
(43) Date of publication of application: **28.04.2021**
(21) Application number: 19204293.5
(22) Date of filing: 21.10.2019
(51) Int. Cl.: C07D 301/12

(54) **PROCESS FOR THE EPOXIDATION OF PROPENE**

(71) Applicant: Evonik Operations GmbH, 45128 Essen (DE); thyssenkrupp Industrial Solutions AG, 45143 Essen (DE)
(72) Inventor: KIBLER, Matthias, 64367 Mühltal (DE); DIETZ, Hans-Christian, 65795 Hattersheim (DE); HAJDUK, Martin, 63773 Goldbach (DE); WÖLL, Sigrid, 63477 Maintal (DE); WÖLL, Wolfgang, 63477 Maintal (DE); PICHA, Petra, 61381 Friedrichsdorf (DE); BREITENBACH, Uwe, 63584 Gründau (DE); SCHMIDT, Franz, 60386 Frankfurt (DE)
(74) Representative: Evonik Patent Association

(57) **Abstract**

In a process for the continuous epoxidation of propene with hydrogen peroxide, comprising a step a) of continuously reacting propene with hydrogen peroxide in the presence of a titanium zeolite epoxidation catalyst to provide a liquid reaction mixture comprising propene oxide, the amount of molecular oxygen formed in step a) is determined, the titanium zeolite catalyst is regenerated when the molar ratio of molecular oxygen formed in step a) to the hydrogen peroxide fed to step a) exceeds a preset threshold value, and step a) is repeated with the regenerated titanium zeolite.

## Description

### Field of the invention

The present invention relates to a continuous process for the epoxidation of propene with hydrogen peroxide in the presence of a titanium zeolite epoxidation catalyst.

### Background of the invention

The epoxidation of propene with hydrogen peroxide in the presence of a titanium silicalite catalyst is known from EP 0 100 119 A1 and has found technical application in the so called HPPO process.

When the epoxidation is carried out continuously, the titanium zeolite catalyst will gradually lose catalytic activity. A desired hydrogen peroxide conversion can be maintained by gradually increasing the reaction temperature to compensate for the loss in catalyst activity.

A deactivated titanium zeolite catalyst can be regenerated by washing with solvent at elevated temperatures, as described in M. G. Clerici et al., Journal of Catalysis, Vol. 129, pages 159 to 167, JP-A 03-114 53, EP 1 190 770 A and WO 2005/000827, or by thermal regeneration, as described in EP 743 094 A, EP 790 075 A, WO 98/55228 and WO 98/55430.

The prior art teaches to regenerate the titanium zeolite catalyst when catalyst activity or selectivity for propene oxide fall below a desired level.

### Summary of the invention

The inventors of the present invention have now found that an optimum for low average consumption of raw materials needed for epoxidizing propene with hydrogen peroxide and a short proportion of reactor downtime due to catalyst regeneration, compared to reactor use for epoxidation, can be achieved, if catalyst regeneration is carried out when the fraction of hydrogen peroxide decomposed to oxygen in the reaction step exceeds a certain limit, which may be the case before the catalyst activity drops to a level where the prior art processes would carry out catalyst regeneration.

Subject of the invention is therefore a process for the continuous epoxidation of propene with hydrogen peroxide, comprising
a) continuously reacting propene with hydrogen peroxide in the presence of a titanium zeolite epoxidation catalyst to provide a liquid reaction mixture comprising propene oxide;
b) determining the amount of molecular oxygen formed in step a);
c) regenerating the titanium zeolite catalyst when the molar ratio of molecular oxygen formed in step a) to the hydrogen peroxide fed to step a) exceeds a preset threshold value; and
d) repeating step a) with titanium zeolite regenerated in step c).

### Detailed description of the invention

In step a) of the process of the invention, propene is continuously reacted with hydrogen peroxide in the presence of a titanium zeolite epoxidation catalyst to provide a liquid reaction mixture comprising propene oxide.

Propene is preferably used in a molar excess to hydrogen peroxide, preferably at a molar ratio of propene to hydrogen peroxide of from 1.1:1 to 30:1, more preferably 2:1 to 10:1 and most preferably 3:1 to 5:1. In a preferred embodiment, propene is used in an excess sufficient to maintain an additional liquid phase rich in propene throughout step a). The propene may contain propane, preferably with a molar ratio of propane to propene of from 0.001 to 0.15 and more preferably of from 0.08 to 0.12.

Hydrogen peroxide can be used as an aqueous solution, preferably containing from 30 to 75 % by weight hydrogen peroxide and most preferably from 40 to 70 % by weight. The aqueous hydrogen peroxide solution is preferably made by an anthraquinone process.

The reaction of propene with hydrogen peroxide can be carried out in the absence or in the presence of a solvent and is preferably carried out in the presence of a solvent. Suitable are all solvents which are not oxidized or are oxidized to only a small extent by hydrogen peroxide under the reaction conditions chosen and which dissolve in water in an amount of more than 10 % by weight. Solvents which are completely miscible with water are preferred. Particularly suitable solvents are alcohols, such as methanol, ethanol or tert-butanol; glycols, such as ethylene glycol, 1,2-propanediol or 1,3-propanediol; cyclic ethers, such tetrahydrofuran, dioxane or propylene oxide; glycol ethers, such ethylene glycol monomethyl ether, ethylene glycol monoethyl ether, ethylene glycol monobutyl ether or the propylene glycol monomethyl ethers; ketones, such as acetone or 2-butanone; and nitriles, such as acetonitrile and propionitrile. When a titanium silicalite is used as epoxidation catalyst, the propene is preferably reacted with hydrogen peroxide in a methanol solvent. The methanol solvent can be a technical grade methanol, a solvent stream recovered in the work-up of the epoxidation reaction mixture or a mixture of both. The methanol solvent may comprise other solvents in minor amounts, such as ethanol, with the amount of such other solvents preferably being less than 2 % by weight. The methanol solvent may also comprise water, preferably from 2 to 13 % by weight water. The solvent is preferably used in the epoxidation in a weight ratio of 0.5 to 20 relative to the combined weight of water and hydrogen peroxide.

The epoxidation catalyst used in step a) preferably comprises a titanium zeolite containing titanium atoms on silicon lattice positions. Preferably, a titanium silicalite catalyst is used, preferably with an MFI or MEL crystal structure. Most preferably a titanium silicalite-1 catalyst with MFI structure as known from EP 0 100 119 A1, is used. The titanium silicalite catalyst is preferably employed as a shaped catalyst in the form of granules, extrudates or shaped bodies. For the shaping process the catalyst may contain 1 to 99% of a binder or carrier material, all binders and carrier materials being suitable that do not react with hydrogen peroxide or with propene oxide under the reaction conditions employed for the epoxidation, silica being preferred as binder. Extrudates with a diameter of 1 to 5 mm are preferably used as shaped catalysts. The amount of catalyst employed may be varied within wide limits and is preferably chosen so that a hydrogen peroxide consumption of more than 90%, preferably more than 95%, is achieved within 1 minute to 5 hours under the employed epoxidation reaction conditions.

The epoxidation reaction of step a) is preferably carried out at a temperature of 20 to 80°C, more preferably at 25 to 60°C. The epoxidation reaction is preferably carried out at a pressure that is higher than the vapor pressure of propene at the reaction temperature in order to maintain the propene dissolved in the solvent or present as a separate liquid phase. The pressure in step a) is preferably from 1.9 to 5.0 MPa, more preferably 2.1 to 3.6 MPa and most preferably 2.4 to 2.8 MPa. Using an excess of propene at a high pressure provides high reaction rate and hydrogen peroxide conversion and at the same time high selectivity for propene oxide.

The epoxidation reaction is preferably carried out with addition of ammonia to improve epoxide selectivity as described in EP 0 230 949 A2. Ammonia is preferably added with a weight ratio of ammonia to the initial amount of hydrogen peroxide of from 0.0001 to 0.003.

The epoxidation reaction of step a) is preferably carried out in a fixed bed reactor by passing a mixture comprising propene, hydrogen peroxide and solvent over a fixed bed comprising a shaped titanium zeolite catalyst. The fixed bed reactor is preferably a tube bundle reactor and the catalyst fixed bed is arranged inside the reactor tubes. The fixed bed reactor is preferably equipped with cooling means and cooled with a liquid cooling medium. The temperature profile along the length of the catalyst fixed bed is preferably adjusted to keep the reaction temperature along 70 to 98 %, preferably along 80 to 95 %, of the length of the catalyst fixed bed within a range of less than 5 °C, preferably within a range of from 0.5 to 3 °C. The temperature of the cooling medium fed to the cooling means is preferably adjusted to a value 3 to 13 °C lower than the maximum temperature in the catalyst fixed bed. The epoxidation reaction mixture is preferably passed through the catalyst bed in down flow mode, preferably with a superficial velocity from 1 to 100 m/h, more preferably 5 to 50 m/h, most preferred 5 to 30 m/h. The superficial velocity is defined as the ratio of volume flow rate/cross section of the catalyst bed. Additionally it is preferred to pass the reaction mixture through the catalyst bed with a liquid hourly space velocity (LHSV) from 1 to 20 h⁻¹, preferably 1.3 to 15 h⁻¹. It is particularly preferred to maintain the catalyst bed in a trickle bed state during the epoxidation reaction. Suitable conditions for maintaining the trickle bed state during the epoxidation reaction are disclosed in WO 02/085873 on page 8 line 23 to page 9 line 15. The epoxidation reaction is most preferably carried out with a catalyst fixed bed maintained in a trickle bed state at a pressure close to the vapor pressure of propene at the reaction temperature, using an excess of propene that provides a reaction mixture comprising two liquid phases, a solvent rich phase and a propene rich liquid phase. Two or more fixed bed reactors may be operated in parallel or in series in order to be able to operate the epoxidation process continuously when regenerating the epoxidation catalyst.

In a side reaction to the reaction of propene with hydrogen peroxide, a part of the hydrogen peroxide fed to step a) is decomposed to molecular oxygen according to the following equation:

2 H₂O₂ → 2 H₂O + O₂

The molecular oxygen formed by decomposition of hydrogen peroxide may remain dissolved in the liquid reaction mixture formed in step a). A part or all of the molecular oxygen formed by decomposition of hydrogen peroxide may also transfer from the liquid reaction mixture into a gas phase present in step a). Molecular oxygen dissolved in the liquid reaction mixture formed in step a) may also be released into a gas phase during work-up of the liquid reaction mixture.

In step b) of the process of the invention, the amount of molecular oxygen formed in step a) is determined. The amount of molecular oxygen may be determined with any method known from the prior art for determining a concentration of molecular oxygen. The amount of molecular oxygen present in a liquid phase, such as the liquid reaction mixture formed in step a), may be determined by gas chromatography from a sample of the liquid phase. Alternatively, a sensor for measuring dissolved oxygen may be used to measure a concentration of oxygen in a liquid phase, such as a polarographic or galvanic electrochemical sensor or a sensor detecting fluorescence quenching by triplet oxygen. The amount of molecular oxygen present in a gas phase may be determined by gas chromatography from a sample of the gas phase, by Raman spectroscopy or by measuring magnetic susceptibility. These methods may be combined for determining the amount of molecular oxygen formed in step a).

In a preferred embodiment, an inert gas stream is introduced in step a) in order to prevent the formation of an oxygen rich, flammable gas phase. A purge gas stream comprising oxygen is obtained from step a), and step b) comprises measuring the flow rate and the oxygen content of said purge gas stream. The oxygen content measured for the purge gas stream may be used to adjust the amount of inert gas introduced in step a) in order to prevent formation of an oxygen rich, flammable gas phase with a minimum amount of inert gas. The inert gas is preferably selected from nitrogen, argon, oxygen depleted air, methane and mixtures thereof and is most preferably nitrogen. Solvent used in step a) and propene oxide product may be removed from the purge gas stream by condensation before the flow rate and the oxygen content of the purge gas stream are measured.

In another preferred embodiment, a work-up gas stream comprising oxygen is separated from the liquid reaction mixture of step a) during work-up of the reaction mixture, preferably by depressurizing the reaction mixture or by distilling propene from the reaction mixture. Step b) then comprises measuring the flow rate and the oxygen content of the work-up gas stream. An inert gas may be introduced during the work-up in order to prevent the work-up gas stream to become oxygen rich and flammable. The same inert gases may be used as in the embodiment described in the preceding paragraph. Solvent used in step a) and propene oxide product may be removed from the work-up gas stream by condensation before the flow rate and the oxygen content of the work-up gas stream are measured. This embodiment and the embodiment described in the preceding paragraph may be combined by combining the purge gas stream with the work-up gas stream and measuring the flow rate and the oxygen content of the resulting combined gas stream, which allows for determining the amount of molecular oxygen formed in step a) with a single measurement.

In yet another preferred embodiment, the liquid reaction mixture of step a) is stripped with an inert gas to provide a strip gas stream and step b) comprises measuring the flow rate and the oxygen content of the strip gas stream. The inert gas is preferably selected from nitrogen, argon, oxygen depleted air, methane and mixtures thereof and is most preferably nitrogen. Stripping is preferably carried out in a stripping column, more preferably in a counter-current stripping column, in order to provide a stripped liquid reaction mixture which is essentially free of oxygen and preferably contains less than 300 ppm by weight oxygen. Stripping is preferably carried out at a temperature below the boiling point of propene at the pressure used in the stripping step in order to prevent propene from distilling off during stripping. The amount of inert gas is preferably selected to provide an oxygen concentration in the strip gas stream in the range of from 0.1 to 10 % by weight, preferably of from 0.5 to 8 % by weight. Preferably, the oxygen concentration in the strip gas stream is measured continuously and the measured value is used to control the amount of inert gas used for stripping in order to achieve an essentially complete stripping of oxygen with a minimum amount of strip gas. Stripping oxygen from the liquid reaction mixture of step a) facilitates further work-up of the reaction mixtures, as no extra safety measures for preventing the formation of flammable gas mixtures are needed in the work-up of the liquid mixture reaction mixture downstream of the stripping. In a further preferred embodiment, an additional inert gas stream is introduced in step a), a purge gas stream comprising oxygen is obtained from step a), this purge gas stream is combined with the strip gas stream, and step b) comprises measuring the flow rate and the oxygen content of the resulting combined gas stream.

In a preferred embodiment, the reaction of step a) is carried out at a pressure of from 0.5 to 50 MPa, preferably from 1.9 to 5.0 MPa, and step b) comprises determining the content of oxygen in a vapor sample obtained by depressurizing liquid reaction mixture of step a). This allows determining the amount of oxygen formed in step a) without a need for measuring the oxygen content of a pressurized liquid and prevents errors in oxygen determination caused by residual hydrogen peroxide and by organic peroxide by-products contained in the liquid reaction mixture. The amount of oxygen contained in the liquid reaction mixture can be determined correctly by one of the following two preferred embodiments. In the first embodiment, step b) comprises a step b1) of withdrawing a sample of the liquid reaction mixture and determining the weight of the sample; a step b2) of depressurizing this sample to provide a depressurized liquid sample and a vapor sample and determining the weight of the depressurized liquid sample, of the vapor sample or of both samples; a step b3) of determining the content of oxygen in the vapor sample; and a step of calculating the content of oxygen in the liquid reaction mixture from the content of oxygen determined in step b3), the weight of the depressurized liquid sample, the vapor sample or both determined in step b2), and the weight of the sample of the liquid reaction mixture determined in step b1). In the second embodiment, step b) comprises a step b1) of withdrawing a sample stream of the liquid reaction mixture and determining the mass flow of the sample stream; a step b2) of depressurizing the sample stream to provide a depressurized liquid sample stream and a vapor sample stream and determining the mass flow of the depressurized liquid sample stream, of the vapor sample stream or of both; a step b3) of determining the content of oxygen in the vapor sample stream; and a step of calculating the content of oxygen in the liquid reaction mixture from the content of oxygen determined in step b3), the mass flow of the depressurized liquid sample stream, the vapor sample stream or both determined in step b2), and the mass flow of the sample stream of the liquid reaction mixture determined in step b1).

In step c) of the process of the invention, the titanium zeolite catalyst is regenerated when the molar ratio of molecular oxygen formed in step a) to the hydrogen peroxide fed to step a) exceeds a preset threshold value. The threshold value is preferably set to a value in the range of from 0.01 to 0.1, more preferably to a value in the range of from 0.04 to 0.08.

The titanium zeolite catalyst can be regenerated by calcination, by treatment with a heated gas, preferably an oxygen containing gas, by treatment with an oxidant in the absence of propene, preferably with hydrogen peroxide, or by a solvent wash. The titanium zeolite catalyst is preferably regenerated by washing it with a solvent, preferably at a temperature of from 80 to 250 °C, more preferably at a temperature of from 100 to 200 °C. The solvent used for regenerating the titanium zeolite catalyst is preferably selected from alcohols, glycols, cyclic ethers, glycol ethers, ketones, nitriles and combinations thereof and preferably comprises methanol, most preferably in an amount of from 50 to 100 % by weight. Regeneration with a methanol solvent is preferably carried out as described in WO 2005/000827. When a solvent is used in step a), the titanium zeolite catalyst is preferably regenerated by washing it with the same solvent as used in step a). When a fixed bed reactor is used in step a) of the process, the regeneration of the titanium zeolite catalyst is preferably carried out without removing it from the fixed bed reactor.

Different methods of regeneration may be combined. Preferably, the catalyst is regenerated by washing with a solvent until the time span for which the catalyst can be used after regeneration has dropped below a desired level. The catalyst is then regenerated by calcination or by treatment with a heated gas. Preferably, the titanium zeolite epoxidation catalyst is regenerated by thermal treatment at 80 to 600 °C, more preferably at 250 to 550 °C, with a gas stream containing oxygen, when the time span for which the catalyst can be used between two regenerations by washing it with an organic solvent falls below a preset threshold value.

In step d) of the process of the invention, the reaction of step a) is repeated with the titanium zeolite catalyst that has been regenerated in step c). The reaction of step a) is preferably repeated at the same reaction conditions. The sequence of steps a) to d) can be repeated as long as the catalyst regains sufficient catalytic activity by the regeneration and the regeneration causes the decomposition of hydrogen peroxide to drop to a level where the molar ratio of molecular oxygen formed in step a) to the hydrogen peroxide fed to step a) is below the preset threshold value. When regeneration cannot restore catalytic activity to the level needed or cannot reduce decomposition of hydrogen peroxide to below the threshold value, the titanium zeolite catalyst needs to be replaced.

Monitoring the amount of molecular oxygen formed in a continuous reaction of propene with hydrogen peroxide and regenerating the titanium catalyst used in the epoxidation when the formation of molecular oxygen exceeds a threshold value allows for maintaining a high yield of propene oxide on hydrogen peroxide and for adjusting an optimum balance between low average consumption of raw materials needed for producing propene oxide and a short proportion of reactor downtime due to catalyst regeneration.

In a preferred embodiment of the process of the invention, the conversion of hydrogen peroxide is kept within a preset range in step a) by increasing the reaction temperature to compensate for activity loss by catalyst deactivation. When the reaction temperature exceeds a predetermined threshold value, the titanium zeolite catalyst is regenerated and step a) is repeated with the regenerated catalyst. In this embodiment, two different criteria for initiating regeneration of the catalysts are applied, i.e. the titanium zeolite catalyst is regenerated if the formation of molecular oxygen by hydrogen peroxide decomposition exceeds a threshold value or if the reaction temperature needed for maintaining the desired conversion of hydrogen peroxide exceeds a threshold value. This embodiment has the advantage of maintaining an essentially constant production capacity for propene oxide and a high yield of propene oxide on hydrogen peroxide. The conversion of hydrogen peroxide is preferably kept within a range of from 90 to 99.9 % and is more preferably kept constant with a deviation from a preset value of less than 2 %. The titanium zeolite catalyst is preferably regenerated when the reaction temperature exceeds a preset value in the range of 50 to 70 °C.

Preferably, a combination of regenerating the titanium zeolite catalyst by washing with a solvent and regenerating the titanium zeolite catalyst by a thermal treatment is used. The time span, for which a regenerated catalyst can be used after regeneration by washing with a solvent before another regeneration has to be carried out, will gradually decrease when the regeneration by washing with a solvent is repeated. Regenerating by thermal treatment is therefore preferably carried out when the time span, for which a regenerated catalyst can be used after regeneration by washing with a solvent has decreased to from 10 to 50 %, more preferably from 30 to 50 %, of the time span for which the fresh catalyst could be used until regeneration becomes necessary. Regeneration by thermal treatment is then preferably carried out at 80 to 600 °C, more preferably at 250 to 500 °C, with a gas stream containing oxygen. After regeneration by thermal treatment, the regenerated catalyst can be used for a longer time span until loss of catalytic activity requires another regeneration. Regeneration can then be further carried out by a sequence of several regenerations by washing with a solvent, followed by thermal regeneration when the time span for which a regenerated catalyst can be used has decreased again to a value of from 10 to 50 %, preferably from 30 to 50 %, of the time span for which the catalyst regenerated by thermal treatment could be used.

### Example

A continuous epoxidation of propene was carried out in a test reactor using a reaction tube with a cooling jacket. The reaction tube had an internal diameter of 30.5 mm and contained a catalyst fixed bed of 10 I of an extruded titanium silicalite catalyst. A mixture of 40 % by weight of propene, 7.7 % by weight of hydrogen peroxide, 3.3 % by weight of water, 49 % by weight of methanol and 77 ppm by weight ammonia was fed to the top of the reaction tube and passed through the catalyst fixed bed in trickle mode. The pressure in the reactor was maintained at 2.6 MPa by introducing nitrogen and the temperature in the reactor was adjusted by controlling coolant flow to maintain an essentially constant conversion of hydrogen peroxide of 97 to 98 %, compensating activity loss of the catalyst by increasing the reaction temperature. The liquid reaction mixture leaving the reaction tube was depressurized to ambient pressure. The flow rate and the oxygen content of the gas phase formed upon depressurizing were determined and the fraction of hydrogen peroxide decomposed to oxygen was calculated from these measurements. The liquid phase remaining after depressurizing was analyzed for propene oxide and hydrogen peroxide and propene oxide yield and hydrogen peroxide conversion were calculated from the analysis results.

After 2335 h of operation, the epoxidation reaction was interrupted by stopping the feed and the catalyst was regenerated within the reactor by passing methanol through the catalyst fixed bed at a rate of 6 kg/h, increasing the reactor temperature to 150 °C at a rate of 10 K/h, maintaining a temperature of 150 °C for 24 h and then decreasing reactor temperature to about 25 °C at a rate of 10 K/h. Epoxidation was then recommenced as described above and continued for another 1155 h. Regeneration was repeated and epoxidation was recommenced again and continued for a further 1094 h. The catalyst was then regenerated a third time as described above, followed by a thermal treatment with a gas containing oxygen. Thermal treatment was carried out by draining the methanol solvent, passing a flow of 68 l/h of nitrogen and 22 l/h of air through the catalyst bed to establish an oxygen concentration of 5 % by volume, heating the reactor at a rate of 10 K/h to a temperature of 280 °C and maintaining this temperature until oxygen consumption by thermal regeneration had ceased and the oxygen concentration of the gas leaving the reactor had risen to the level of the gas entering the reactor. The reactor was then cooled to room temperature at 10 K/h maintaining the gas flow. Thereafter, the gas supply was stopped, the catalyst was rewetted as described in WO 2016/016070 by passing water through the catalyst fixed bed and then increasing the methanol content in the liquid passed through the catalyst fixed bed stepwise up to the level used during epoxidation before epoxidation was recommenced a third time.

Table 1 summarizes the changes in propene oxide yield, hydrogen peroxide decomposition and average temperature in the catalyst fixed bed over the course of the epoxidation reaction. In the first 150 to 200 h after starting or recommencing the epoxidation reaction, hydrogen peroxide decomposition decreased and propene oxide yield in general increased. From then on, hydrogen peroxide decomposition slowly increased and propene oxide yield slowly decreased until regeneration became necessary.

**Table 1**

| Total run time in h | Run time since last regeneration in h | Propene oxide yield on H₂O₂ in % | Hydrogen peroxide decomposition in % | Average temperature in catalyst fixed bed in °C |
|---|---|---|---|---|
| 50 | | 82.9 | 7.5 | 29.6 |
| 150 | | 85.8 | 5.4 | 34.2 |
| 1100 | | 84.2 | 6.7 | 44.5 |
| 2300 | | 79.1 | 8.3 | 52.2 |
| 2385 | 50 | 85.4 | 7.3 | 35.1 |
| 2485 | 150 | 84.4 | 6.7 | 36.5 |
| 3435 | 1100 | 81.5 | 7.7 | 42.4 |
| 3540 | 50 | 83.0 | 6.5 | 34.0 |
| 3640 | 150 | 85.2 | 6.0 | 38.6 |
| 4560 | 1070 | 82.0 | 7.1 | 55.1 |
| 4634 | 50 | 83.9 | 6.7 | 32.3 |
| 4734 | 150 | 85.7 | 5.5 | 34.8 |
| 5684 | 1100 | 83.5 | 6.3 | 50.1 |

## Claims

1. A process for the continuous epoxidation of propene with hydrogen peroxide, comprising
a) continuously reacting propene with hydrogen peroxide in the presence of a titanium zeolite epoxidation catalyst to provide a liquid reaction mixture comprising propene oxide;
b) determining the amount of molecular oxygen formed in step a);
c) regenerating the titanium zeolite catalyst when the molar ratio of molecular oxygen formed in step a) to the hydrogen peroxide fed to step a) exceeds a preset threshold value; and
d) repeating step a) with titanium zeolite regenerated in step c).

2. The process of claim 1, wherein said threshold value is in the range of from 0.01 to 0.1.

3. The process of claim 1 or 2, wherein an inert gas stream is introduced in step a), a purge gas stream comprising oxygen is obtained from step a), and step b) comprises measuring the flow rate and the oxygen content of said purge gas stream.

4. The process of claim 1 or 2, wherein a work-up gas stream comprising oxygen is separated from the liquid reaction mixture of step a) by depressurizing said reaction mixture or by distilling propene from said reaction mixture and step b) comprises measuring the flow rate and the oxygen content of said work-up gas stream.

5. The process of claim 4, wherein an inert gas stream is introduced in step a), a purge gas stream comprising oxygen is obtained from step a), the purge gas stream is combined with said work-up gas stream, and step b) comprises measuring the flow rate and the oxygen content of the resulting combined gas stream.

6. The process of claim 1 or 2, wherein the liquid reaction mixture of step a) is stripped with an inert gas to provide a strip gas stream and step b) comprises measuring the flow rate and the oxygen content of said strip gas stream.

7. The process of claim 6, wherein an inert gas stream is introduced in step a), a purge gas stream comprising oxygen is obtained from step a), the purge gas stream is combined with said strip gas stream, and step b) comprises measuring the flow rate and the oxygen content of the resulting combined gas stream.

8. The process of any one of claims 1 to 3, wherein step a) is carried out at a pressure of from 0.5 to 50 MPa and step b) comprises
b1) withdrawing a sample of said liquid reaction mixture and determining the weight of said sample;
b2) depressurizing said sample to provide a liquid sample and a vapor sample and determining the weight of said liquid sample, said vapor sample or both;
b3) determining the content of oxygen in said vapor sample;
b4) calculating the content of oxygen in said liquid reaction mixture from the content of oxygen determined in step b3), the weight of said liquid sample, said vapor sample or both determined in step b2), and the weight of said sample of the liquid reaction mixture determined in step b1).

9. The process of any one of claims 1 to 3, wherein step a) is carried out at a pressure of from 0.5 to 50 MPa and step b) comprises
b1) withdrawing a sample stream of said liquid reaction mixture and determining the mass flow of said sample stream;
b2) depressurizing said sample stream to provide a liquid sample stream and a vapor sample stream and determining the mass flow of said liquid sample stream, said vapor sample stream or both;
b3) determining the content of oxygen in said vapor sample stream;
b4) calculating the content of oxygen in said liquid reaction mixture from the content of oxygen determined in step b3), the mass flow of said liquid sample stream, said vapor sample stream or both determined in step b2), and the mass flow of said sample stream of the liquid reaction mixture determined in step b1).

10. The process of any one of claims 1 to 9, wherein the amount of oxygen is determined by Raman spectroscopy, by gas chromatography, by measurement of magnetic susceptibility or by a combination thereof.

11. The process of any one of claims 1 to 10, wherein in step a) the conversion of hydrogen peroxide is kept within a preset range by increasing the reaction temperature to compensate for activity loss by catalyst deactivation, the titanium zeolite catalyst is regenerated when the reaction temperature exceeds a predetermined threshold value, and step a) is repeated with the regenerated catalyst.

12. The process of claim 11, wherein the conversion of hydrogen peroxide is kept within a range of from 90 to 99.9 % and the titanium zeolite catalyst is regenerated when the reaction temperature exceeds a preset value in the range of 50 to 70 °C.

13. The process of claim 12, wherein the conversion of hydrogen peroxide is kept constant with a deviation from a preset value of less than 2 %.

14. The process of any one of claims 1 to 13, wherein the titanium zeolite epoxidation catalyst is regenerated by washing it with a solvent, preferably at a temperature of from 80 to 250 °C.

15. The process of claim 14, wherein a solvent is used in step a) and said catalyst is regenerated by washing it with the solvent used in step a).

16. The process of claim 14 or 15, wherein said solvent is selected from alcohols, glycols, cyclic ethers, glycol ethers, ketones, nitriles and combinations thereof.

17. The process of claim 16, wherein said solvent comprises methanol, preferably in an amount of from 50 to 100 % by weight.

18. The process of any one of claims 14 to 17, wherein the titanium zeolite epoxidation catalyst is additionally regenerated by thermal treatment at 80 to 600 °C with a gas stream containing oxygen if regeneration by washing with a solvent no longer restores catalyst activity for a desired time span.
